# EUROPÄISCHE PATENTSCHRIFT

(11) **EP 2 633 825 B1**
(45) Veröffentlichungstag und Bekanntmachung des Hinweises auf die Patenterteilung: **14.06.2017**
(21) Anmeldenummer: 13166590.3
(22) Anmeldetag: 04.11.2008
(51) Int. Cl.: A61B 17/32, A61B 17/3203, A61M 5/14, A61B 90/00

(54) **Wasserstrahlchirurgiegerät und Verfahren zum Betrieb eines solchen**
Water jet surgical device and method for operating same
Appareil chirurgical à jet d'eau et procédé de fonctionnement d'un tel système

(30) Priorität: 06.11.2007 DE 102007052805
(43) Veröffentlichungstag der Anmeldung: 04.09.2013
(62) Teilanmeldung aus: 08848108.0
(73) Patentinhaber: Erbe Elektromedizin GmbH, 72072 Tübingen (DE)
(72) Erfinder: Wahl, Hans-Jürgen, 72818 Trochtelfingen (DE); Pfäffle, Alexander, 72810 Gomaringen (DE); Kühner, Ralf, 70567 Stuttgart (DE)
(74) Vertreter: Bohnenberger, Johannes

(56) Entgegenhaltungen:
- EP-A- 0 879 578
- US-A- 4 655 742
- US-A- 5 505 729
- US-A- 5 536 242
- US-A1- 2005 059 924
- US-A1- 2006 156 875
- US-A1- 2006 247 743

## Beschreibung

Die Erfindung betrifft ein Wasserstrahlchirurgiegerät nach dem Oberbegriff des Patentanspruches 1 sowie ein Verfahren zum Betreiben eines solchen.

Wasserstrahlchirurgiegeräte, mittels derer Gewebe durch einen Hochdruck-Wasserstrahl getrennt werden kann, sind allgemein bekannt. Wenn man ein solches Gerät in der offenen Chirurgie benutzt, so ist das Abführen der "Schneidflüssigkeit" unproblematisch. Bei Verwendung derartiger Geräte bei endoskopischen Operationen, also innerhalb von Körperhöhlen, können durch die Zufuhr der Schneidflüssigkeit Probleme auftreten.

Die EP 0 879 578 A beschreibt einen Apparat zum Einbringen eines flüssigen, Thrombose lösenden Medikaments unter Druck in ein Gefäß und von hier in den Körper. Der Apparat weist Sensoren auf um den Druck und das Bolusvolumen zu überwachen. In der Druckschrift wird das Problem der Verfälschung der abgegebenen Menge an Flüssigkeit durch die Füllmenge der Leitung nicht erwähnt.

Die US 2005/059924 A beschreibt eine Vorrichtung, um einem Patienten kontrolliert Medikamente zuzuführen. Somit beschäftigt sich auch diese Druckschrift lediglich mit der Verabreichung von Medikamenten.

Die US 2006/0156875 A1 beschreibt ein gattungsbildenes Wasserstrahlschneidgerät. Die Menge der abgegebenen Flüssigkeit wird in der Druckschrift nicht thematisiert.

Die US 2006/0247743 A1 beschreibt ebenfalls ein Wasserstrahlgerät, wobei jedoch ein lichtabsorbierendes Fluid mit Hilfe eines Laserstrahls ausgetrieben wird. Für die Bestimmung einer tatsächlich abgegebenen Menge an Flüssigkeit stellt dieses Dokument keine Lösung bereit.

Ausgehend von der US 2006/0156875 A1 ist es Aufgabe der vorliegenden Erfindung, ein Wasserstrahlchirurgiegerät bereitzustellen mittels dessen bei endoskopischen Operationen auftretende Probleme vermieden werden können.

Diese Aufgabe wird durch ein Wasserstrahlchirurgiegerät nach Anspruch 1 bzw. ein Verfahren nach Anspruch 9 gelöst.

Die Messeinrichtung umfasst vorzugsweise einen Füllsensor und erzeugt ein Füllsignal dann, wenn die Anschlussleitung des Chirurgieinstruments im Wesentlichen bis zur Auslassdüse gefüllt ist und gibt eine zum Füllen der Anschlussleitung benötigte Füllmenge wieder. Auf diese Weise kann das Wasserstrahlchirurgiegerät in dem Sinne funktionsbereit gemacht werden, dass der Operateur dann, wenn er ein Startsignal zum Beginn eines Schneidvorgangs gibt, tatsächlich Schneidflüssigkeit an der Auslassdüse ansteht.

Vorzugsweise ist die Anzeige- oder Registriereinheit derart ausgebildet, dass die Menge abgegebener Flüssigkeit abzüglich der Füllmenge angezeigt wird. Diese Füllmenge kann besonders bei langen Anschlussleitungen oder Chirurgieinstrumenten mit einer hohen Flüssigkeitskapazität relativ beträchtlich sein, sodass die Messung der in den Körper abgegebenen Flüssigkeitsmenge verfälscht wird.

Vorzugsweise ist eine Eingabevorrichtung vorgesehen, über welche die Steuereinrichtung ein dem angeschlossenen Chirurgieinstrument entsprechendes Mengensignal zur Abgabe einer von der Flüssigkeitsfördereinrichtung abzugebenden Flüssigkeitsmenge zuführbar ist. Bei dieser Ausführungsform der Erfindung muss also nicht unbedingt gemessen werden, wann die Anschlussleitung bzw. das Chirurgieinstrument befüllt ist, es wird hier vielmehr eine Menge von Schneidflüssigkeit vorgegeben, die von der Flüssigkeitsfördereinrichtung abgegeben wird, sodass man sich dann sicher sein kann, dass das Gerät befüllt ist.

Diese Eingabe der Flüssigkeitsmenge kann durch eine manuell bedienbare Eingabevorrichtung vorgegeben werden. Bei einer anderen bevorzugten Ausführungsform der Erfindung wird die zum Befüllen des Chirurgieinstrumentes bzw. seiner Anschlussleitung notwendige Flüssigkeitsmenge direkt durch eine Codiereinrichtung im Chirurgieinstrument der Eingabevorrichtung übermittelt, sodass ein manuelles Programmieren entfällt.

Wenn ein Füllsensor vorhanden ist, so kann dieser als Drucksensor ausgebildet sein, der bei einer vorbestimmten Druckänderung oder einem vorbestimmten zeitlichen Verlauf des Druckes das Füllsignal erzeugt. Der Druck der Schneidflüssigkeit ist nämlich niedrig, solange aus der Auslassdüse Luft herausgedrückt wird.

Bei einer anderen Ausführungsform der Erfindung umfasst die Sensoreinrichtung einen Feuchtigkeits- oder Leitfähigkeitssensor, der vorzugsweise sehr nahe an der Auslassdüse angebracht ist und dann ein Signal erzeugt, wenn die Flüssigkeit tatsächlich an diesem Ort angekommen ist.

Verfahrensmäßig umfasst das Verfahren zum Betreiben eines Wasserstrahlchirurgiegerätes die folgenden Schritte:
- Anschließen eines Chirurgieinstruments an das Wasserstrahlchirurgiegerät;
- Erzeugen von Ansteuersignalen zum Ansteuern einer Flüssigkeitsfördereinrichtung derart, dass diese Flüssigkeit solange dem Chirurgieinstrument zuführt, bis dieses im Wesentlichen bis zu seiner Auslassdüse gefüllt ist;
- Beenden der Ansteuersignale und Erzeugen eines Betriebsfreigabesignals derart, dass eine Ansteuerung der Flüssigkeitsfördereinrichtung zum Operationsbetrieb des Wasserstrahlchirurgiegeräts zugelassen wird.

Es wird also in diesem Fall eine Betätigung des Wasserstrahlchirurgiegeräts unter Operationsbedingungen verhindert, bis das Chirurgieinstrument wirklich "startklar" ist.

Nachfolgend wird eine Ausführungsform der Erfindung anhand von Abbildungen näher erläutert. Hierbei zeigen
- Fig. 1: eine schematische Darstellung zur Erläuterung der verschiedenen Baugruppen und
- Fig. 2: ein Diagramm zur Erläuterung des Druckverlaufes beim Befüllen des Chirurgieinstruments.

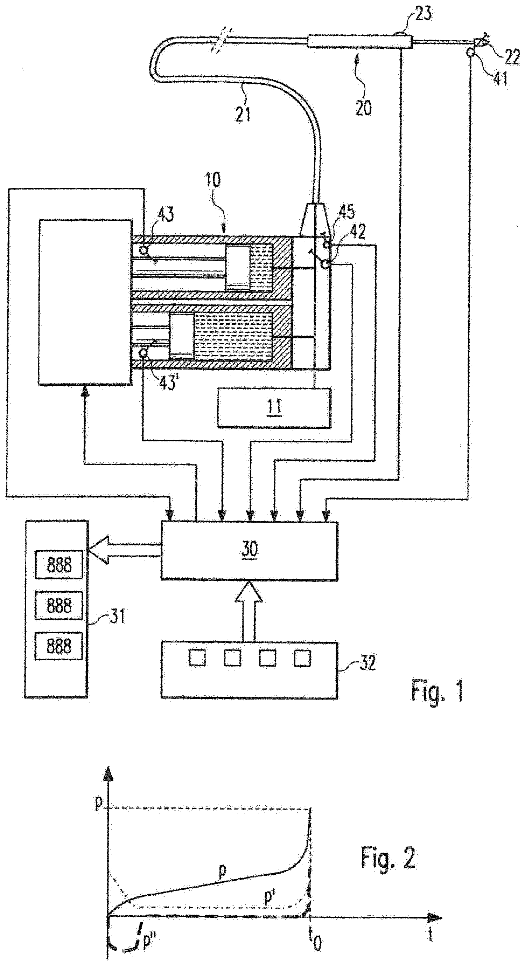

In Fig. 1 ist als Flüssigkeitsfördereinrichtung 10 eine Pumpe mit zwei Kolben dargestellt, die aus einem Vorratsbehälter 11 Schneidflüssigkeit über eine Anschlussleitung 21 eines Chirurgieinstrumentes 20 bis zu einer Auslassdüse 22 fördert, aus welcher bei Betätigung einer Bedienungstaste 23 Schneidflüssigkeit aus der Auslassdüse 22 zum Trennen von Gewebe oder auch zum Injizieren in ein Gewebe ausgestoßen wird. Selbstverständlich sind auch andere Druckerzeugungseinrichtungen verwendbar.

Die Kolben der bei diesem Ausführungsbeispiel gezeigten Flüssigkeitsfördereinrichtung werden hinsichtlich ihrer Position über Positionssensoren 43, 43' überwacht, deren Ausgangssignale einer Steuereinrichtung 30 zugeführt werden.

Weiterhin ist an der Flüssigkeitsfördereinrichtung 10 ein Ankoppelfühler 45 vorgesehen, der dann anspricht, wenn ein Chirurgieinstrument 20 mit der Flüssigkeitsfördereinrichtung 10 verbunden wird.

Der von der Flüssigkeitsfördereinrichtung 10 erzeugte Druck wird mittels eines Drucksensors 42 abgetastet, dessen Ausgangssignale der Steuereinrichtung 30 zugeführt werden. Alternativ oder zusätzlich kann ein Feuchtesensor 41 in der Auslassdüse 22 oder zumindest in deren Nähe vorgesehen sein, der dann anspricht, wenn Schneidflüssigkeit bis zu diesem Punkt gelangt ist.

Die Steuereinrichtung 30 ist mit einer Anzeigeeinheit 31 verbunden, auf welcher die verschiedenen Parameter, insbesondere aber die von der Flüssigkeitsfördereinrichtung 10 geförderte und aus der Auslassdüse 22 ausgestoßene Flüssigkeitsmenge angezeigt wird.

Weiterhin ist eine Eingabevorrichtung 32 vorgesehen, über welche der Steuereinrichtung 30 Betriebsdaten eingegeben werden können, wobei diese Betriebsdaten zum Beispiel die Flüssigkeitsmenge wiedergeben, welche in das Chirurgieinstrument 20 zu fördern ist, wenn dieses bis zu seiner Auslassdüse 22 hin gefüllt werden soll.

Wenn also ein Chirurgieinstrument 20 an die Flüssigkeitsfördereinrichtung 10 angesteckt wird, so erzeugt der Ankoppelfühler 45 ein entsprechendes Signal, welches der Steuereinrichtung 30 mitgeteilt wird. Auf ein entsprechendes "Füllkommando" hin, welches der Operateur über die Eingabevorrichtung 32 eingibt, arbeitet nun die Flüssigkeitsfördereinrichtung 10 solange, bis das Chirurgieinstrument 20 bis zu seiner Auslassdüse 22 hin mit Schneidflüssigkeit gefüllt ist. Danach kann der Operateur das Gerät durch Betätigen der Bedienungstaste 23 aktivieren, also einen Strahl von Schneidflüssigkeit aus der Auslassdüse 22 derart austreten lassen, dass ein anvisiertes Zielgewebe durchtrennt wird. Die während der Operation abgegebene und tatsächlich aus der Auslassdüse 22 ausgetretene Flüssigkeitsmenge, also die Gesamtfördermenge abzüglich der im Chirurgieinstrument 20 enthaltenen Flüssigkeitsmenge wird auf der Anzeigeeinrichtung 31 angezeigt.

In Fig. 2 ist ein Druckverlauf idealisiert dargestellt. Der Druck p steigt nach diesem Diagramm an, während die Schneidflüssigkeit in die Anschlussleitung in Richtung auf die Auslassdüse 22 strömt, da das Luftpolster, welches die Flüssigkeit vor sich her treibt, immer kleiner wird. In dem Moment (t-Index 0), in welchem die Schneidflüssigkeit die Auslassdüse 22 erreicht, steigt der Druck abrupt an, da die Schneidflüssigkeit aufgrund ihrer gegenüber Luft sehr hohen Viskosität einen höheren Strömungswiderstand an der (sehr kleinen) Auslassdüse 22 aufweist. Dieser abrupte Druckanstieg kann dazu benützt werden, ein Signal abzugeben, welches anzeigt, dass nun das Chirurgieinstrument 20 korrekt gefüllt und somit betriebsbereit ist. Noch leichter ist die zweite Ableitung des Druckes nach der Zeit zum Generieren eines solchen Signals geeignet, da diese nur dann einen positiven Wert aufweist, wenn der genannte steile Druckanstieg tatsächlich vorliegt.

### Bezugszeichenliste

- 10: Flüssigkeitsfördereinrichtung
- 11: Vorratsbehälter
- 20: Chirurgieinstrument
- 21: Anschlussleitung
- 22: Auslassdüse
- 23: Bedienungstaste
- 30: Steuereinrichtung
- 31: Anzeigeeinheit
- 32: Eingabevorrichtung
- 41: Feuchtesensor
- 42: Drucksensor
- 43, 43': Positionssensor
- 45: Ankoppelfühler

## Patentansprüche

1. Wasserstrahlchirurgiegerät, umfassend
eine Flüssigkeitsfördereinrichtung (10), die zur Abgabe einer Flüssigkeit in eine Anschlussleitung (21) eines Chirurgieinstruments (20) mit einer Auslassdüse (22) durch Ansteuersignale aus einer Steuereinrichtung (30) ansteuerbar ist,
**dadurch gekennzeichnet, dass**
mindestens eine Messeinrichtung (41 - 43) vorgesehen und derart ausgebildet ist, dass nach einem Anschließen der Flüssigkeitsfördereinrichtung (10) die Messeinrichtung (41 - 43) Messsignale zur Darstellung einer Menge tatsächlich aus der Auslassdüse (22) des Chirurgieinstruments (20) abgegebener Flüssigkeit in einer Anzeige- oder Registriereinheit (31) erzeugt.

2. Wasserstrahlchirurgiegerät nach Anspruch 1,
**dadurch gekennzeichnet, dass**
die Messeinrichtung (41, 42) einen Füllsensor umfasst und ein Füllsignal dann erzeugt, wenn die Anschlussleitung (21) im Wesentlichen bis zur Auslassdüse (22) gefüllt ist und eine zum Füllen der Anschlussleitung (21) benötigte Füllmenge wiedergibt.

3. Wasserstrahlchirurgiegerät nach Anspruch 1 oder 2,
**dadurch gekennzeichnet, dass**
die Anzeige- oder Registriereinheit (31) derart ausgebildet ist, dass die Menge abgegebener Flüssigkeit abzüglich der Füllmenge angezeigt wird.

4. Wasserstrahlchirurgiegerät nach einem der vorhergehenden Ansprüche,
**dadurch gekennzeichnet, dass**
eine Eingabevorrichtung (32) vorgesehen ist, wobei über die Eingabevorrichtung (32) der Steuereinrichtung (30) ein dem Chirurgieinstrument (20) entsprechendes Mengensignal zur Abgabe einer von der Flüssigkeitsfördereinrichtung (10) abzugebenden Flüssigkeitsmenge zuführbar ist

5. Wasserstrahlchirurgiegerät nach Anspruch 4,
**dadurch gekennzeichnet, dass**
die Eingabevorrichtung (32) manuell bedienbar ist.

6. Wasserstrahlchirurgiegerät nach Anspruch 4 oder 5,
**dadurch gekennzeichnet, dass**
die Eingabevorrichtung (32) durch das Chirurgieinstrument (20) programmierbar ist.

7. Wasserstrahlchirurgiegerät nach einem der vorhergehenden Ansprüche, insbesondere nach einem der Ansprüche 2 bis 6,
**dadurch gekennzeichnet, dass**
der Füllsensor einen Drucksensor (42) umfasst, der bei einer vorbestimmten Druckänderung oder einem vorbestimmten zeitlichen Verlauf des Druckes das Füllsignal erzeugt.

8. Wasserstrahlchirurgiegerät nach einem der vorhergehenden Ansprüche, insbesondere nach einem der Ansprüche 2 bis 7,
dadurc**h gekennzeichnet**, dass
der Füllsensor einen Feuchtigkeits- oder Leitfähigkeitssensor (41) umfasst.

9. Verfahren zum Betreiben eines Wasserstrahlchirurgiegerätes, umfassend die Schritte
- Anschließen eines Chirurgieinstruments (20) an das Wasserstrahlchirurgiegerät;
- Erzeugen von Ansteuersignalen zum Ansteuern einer Flüssigkeitsfördereinrichtung derart, dass diese Flüssigkeit so lange dem Chirurgieinstrument (20) zuführt, bis dieses im Wesentlichen bis zu seiner Auslassdüse (22) gefüllt ist;
- Beenden der Ansteuersignale und Erzeugen eines Betriebsfreigabesignals derart, dass eine Ansteuerung der Flüssigkeitsfördereinrichtung zum Operationsbetrieb des Wasserstrahlchirurgiegerätes zugelassen wird;
- Darstellen einer Menge einer tatsächlich aus der Auslassdüse (22) des Chirurgieinstruments (20) abgegebenen Flüssigkeit in einer Anzeige oder Registrieren dieser Menge in einer Registriereinheit (31).

## Claims

1. Water jet surgical device, comprising a liquid feed device (10), which is controllable by drive signals from a control device (30) in order to dispense a liquid into an attachment line (21) of a surgical instrument (20) with an outlet nozzle (22), **characterized in that** at least one measuring device (41-43) is provided and designed in such a way that, after the liquid feed device (10) has been attached, the measuring device (41-43) generates measurement signals in order to show, in a display or registration unit (31), a quantity of liquid actually dispensed from the outlet nozzle (22) of the surgical instrument (20).

2. Water jet surgical device according to Claim 1, **characterized in that** the measuring device (41, 42) comprises a filling sensor and generates a filling signal when the attachment line (21) is filled substantially as far as the outlet nozzle (22) and represents a filling quantity needed to fill the attachment line (21).

3. Water jet surgical device according to Claim 1 or 2, **characterized in that** the display or registration unit (31) is designed in such a way that the quantity of liquid dispensed minus the filling quantity is displayed.

4. Water jet surgical device according to one of the preceding claims, **characterized in that** an input device (32) is provided, wherein a quantity signal corresponding to the surgical instrument (20) can be delivered via the input device (32) to the control device (30) in order to dispense a liquid quantity that is to be dispensed by the liquid feed device (10).

5. Water jet surgical device according to Claim 4, **characterized in that** the input device (32) can be operated manually.

6. Water jet surgical device according to Claim 4 or 5, **characterized in that** the input device (32) is programmable via the surgical instrument (20).

7. Water jet surgical device according to one of the preceding claims, in particular according to one of Claims 2 to 6, **characterized in that** the filling sensor comprises a pressure sensor (42) which generates the filling signal at a predetermined pressure change or a predetermined time profile of the pressure.

8. Water jet surgical device according to one of the preceding claims, in particular according to one of Claims 2 to 7, **characterized in that** the filling sensor comprises a moisture or conductivity sensor (41).

9. Method for operating a water jet surgical device, comprising the steps of
- attaching a surgical instrument (20) to the water jet surgical device;
- generating drive signals in order to drive a liquid feed device in such a way that the latter delivers liquid to the surgical instrument (20) until the latter is filled substantially as far as the outlet nozzle (22) thereof;
- ending the drive signals and generating an operation enable signal in such a way as to permit driving of the liquid feed device for the operating mode of the water jet surgical device;
- showing a quantity of a liquid actually dispensed from the outlet nozzle (22) of the surgical instrument (20) in a display, or registering this quantity in a registration unit (31).

## Revendications

1. Appareil chirurgical à jet d'eau, comprenant
un dispositif de transport de liquide (10), qui peut être commandé pour délivrer un liquide dans une conduite de raccordement (21) d'un instrument chirurgical (20) avec une buse de sortie (22) au moyen de signaux de commande émanant d'un dispositif de commande (30),
**caractérisé en ce qu'**il est prévu au moins un dispositif de mesure (41-43) configuré de telle manière qu'après un raccordement du dispositif de transport de liquide (10) le dispositif de mesure (41-43) produise des signaux de mesure pour la représentation d'une quantité de liquide effectivement délivrée hors de la buse de sortie (22) de l'instrument chirurgical (20) dans une unité d'affichage ou d'enregistrement (31).

2. Appareil de chirurgie à jet d'eau selon la revendication 1, **caractérisé en ce que** le dispositif de mesure (41, 42) comprend un capteur de remplissage et produit un signal de remplissage, lorsque la conduite de raccordement (21) est remplie essentiellement jusqu'à la buse de sortie (22) et reproduit une quantité de remplissage nécessaire pour remplir la conduite de raccordement (21).

3. Appareil de chirurgie à jet d'eau selon la revendication 1 ou 2, **caractérisé en ce que** l'unité d'affichage ou d'enregistrement (31) est configurée de telle manière que la quantité de liquide délivrée soit affichée avec déduction de la quantité de remplissage.

4. Appareil de chirurgie à jet d'eau selon l'une quelconque des revendications précédentes, **caractérisé en ce qu'**il est prévu un dispositif d'entrée (32), dans lequel un signal de quantité correspondant à l'instrument chirurgical (20) pour la délivrance d'une quantité de liquide à délivrer par le dispositif de transport de liquide (10) peut être fourni au dispositif de commande (30) par le dispositif d'entrée (32).

5. Appareil de chirurgie à jet d'eau selon la revendication 4, **caractérisé en ce que** le dispositif d'entrée (32) peut être commandé à la main.

6. Appareil de chirurgie à jet d'eau selon la revendication 4 ou 5, **caractérisé en ce que** le dispositif d'entrée (32) peut être programmé par l'instrument chirurgical (20).

7. Appareil de chirurgie à jet d'eau selon l'une quelconque des revendications précédentes, en particulier selon l'une quelconque des revendications 2 à 6, **caractérisé en ce que** le capteur de remplissage comprend un capteur de pression (42), qui produit le signal de remplissage lors d'une variation de pression prédéterminée ou lors d'une évolution temporelle prédéterminée de la pression.

8. Appareil de chirurgie à jet d'eau selon l'une quelconque des revendications précédentes, en particulier selon l'une quelconque des revendications 2 à 7, **caractérisé en ce que** le capteur de remplissage comprend un détecteur d'humidité ou de conductibilité (41).

9. Procédé d'utilisation d'un appareil de chirurgie à jet d'eau, comprenant les étapes suivantes:
- raccorder un instrument chirurgical (20) à l'appareil de chirurgie à jet d'eau;
- produire des signaux de commande pour commander un dispositif de transport de liquide, de telle manière que ce liquide soit envoyé à l'instrument chirurgical (20) jusqu'à ce que celui-ci soit rempli essentiellement jusqu'à sa buse de sortie (22);
- cesser les signaux de commande et produire un signal d'autorisation de fonctionnement, de telle manière qu'une commande du dispositif de transport de liquide pour le fonctionnement opératoire de l'appareil de chirurgie à jet d'eau soit autorisée;
- représenter une quantité de liquide effectivement délivrée hors de la buse de sortie (22) de l'instrument chirurgical (20) dans un affichage ou un enregistrement de cette quantité dans une unité d'enregistrement (31).
